# EUROPÄISCHE PATENTSCHRIFT

(11) **EP 3 525 769 B1**
(45) Veröffentlichungstag und Bekanntmachung des Hinweises auf die Patenterteilung: **12.05.2021**
(21) Anmeldenummer: 18711039.0
(22) Anmeldetag: 05.03.2018
(51) Int. Cl.: A61K 9/20, A61K 38/16, A61K 9/10, A61K 9/107, A61K 47/46, A61P 1/18

(54) **SCHMELZTABLETTE ENTHALTEND BURLULIPASE UND DARAUS HERGESTELLTE PHARMAZEUTISCHE ZUSAMMENSETZUNG**
ORODISPERSIBLE TABLET COMPRISING BURLULIPASE AND ITS PHARMACEUTICAL COMPOSITION
COMPRIME ORODISPERSIBLE DE BURLULIPASE ET SA COMPOSITION PHARMACEUTIQUE

(30) Priorität: 03.03.2017 DE 102017104472
(43) Veröffentlichungstag der Anmeldung: 21.08.2019
(73) Patentinhaber: Nordmark Pharma GmbH, 25436 Uetersen (DE)
(72) Erfinder: FUHRHERR, Richard, 25436 Uetersen (DE); LÜDEMANN, Jan, 25436 Uetersen (DE); GARRETT, Lisa, Swindon Wiltshire SN5 8RU (GB)
(74) Vertreter: RGTH
(86) Internationale Anmeldenummer: PCT/EP2018/055285
(87) Internationale Veröffentlichungsnummer: WO 2018/158459

(56) Entgegenhaltungen:
- WO-A1-2006/044529
- WO-A1-2010/025126
- WO-A1-2010/085975
- CN-A- 104 109 662
- DE-A1-102009 006 594

## Beschreibung

### Einleitung

Die vorliegende Erfindung betrifft eine Schmelztablette die dadurch gekennzeichnet ist, dass sie Burlulipase umfasst. Sie betrifft ferner flüssige pharmazeutische Zusammensetzungen die Lösungen solcher Schmelztabletten in Wasser oder anderen Getränken enthalten. Sie betrifft Arzneimittel die solche Schmelztabletten oder Lösungen enthalten oder daraus bestehen. Insbesondere betrifft sie solche Arzneimittel die zur Behandlung von Verdauungsproblemen, insbesondere exokriner Pankreasinsuffizienz geeignet sind. Insbesondere sind sie zur Behandlung von exokriner Pankreasinsuffizienz bei Patienten mit Mukoviszidose und zur Behandlung von exokriner Pankreasinsuffizienz von Patienten in der Pädiatrie geeignet.

### Stand der Technik

Exokrine Pankreasinsuffizienz ist eine Krankheit, bei der eine ungenügende Bildung von Bauchspeicheldrüsenenzymen auftritt, wodurch die Nahrung nicht mehr ausreichend aufgeschlossen werden kann. Diese kann beispielsweise durch Verlust von Bauchspeicheldrüsengewebe bei chronischer Pankreatitis oder durch Bauchspeicheldrüsenkrebs erworben, aber auch durch genetisch bedingte Erkrankungen, wie Mukoviszidose, angeboren sein. Die exokrine Pankreasinsuffizienz führt zu verschiedenen Verdauungsproblemen wie Steatorrhoe (Fettdurchfall) und wird in der Regel durch Verabreichung von Verdauungsenzymen mit den Mahlzeiten behandelt (Enzymersatztherapie).

Die Mukoviszidose ist eine autosomal-rezessiv vererbte Stoffwechselerkrankung, bei der die Zusammensetzung aller Sekrete exokriner Drüsen verändert wird. Dabei ist der Salz- und Wassertransport der Zellen gestört und unter anderem sind die von der Bauchspeicheldrüse gebildeten Verdauungssäfte zäher als normal und verstopfen die Ausführungsgänge der Drüse. Die sich anstauenden Verdauungssäfte führen zu einer Reizung und schließlich einer Schädigung der Bauchspeicheldrüse.

Bei der Enzymsubstitution wird ein aus den Bauchspeicheldrüsen von Schweinen gewonnenes Produkt, in Form von sogenanntem "Pankreatin" peroral verabreicht. Pankreatin ist ein Feststoff, der in einem mehrstufigen Verfahren hergestellt wird, das das Entfetten von Bauchspeicheldrüsen von Schweinen und das Entfernen von Fasern daraus umfasst. Es ist ein braunes Pulver, das neben den erwünschten Hauptbestandteilen Lipase, Protease und Amylase noch eine große Anzahl von Rückständen aus den Bauchspeicheldrüsen der Schweine enthält. Pankreatin wird im Europäischen Arzneibuch (Ph. Eur.) als "Pankreas-Pulver" (Pancreas Powder) und im USamerikanischen Arzneibuch (USP) als "Pancreatin" bzw. "Pancrelipase" beschrieben. Allgemein verfügbare Verabreichungsformen für Pankreatin sind überzogene Tabletten, Mikrotabletten, Mikrodragees, Kapseln, Pulver und Granulate bzw. Pellets, bevorzugt mit magensaftresistentem Filmüberzug, da die Pankreasenzyme, insbesondere die Pankreatin-Lipase, im sauren Magensaft instabil sind.

Die Behandlung mit Pankreatin hat jedoch Nachteile. Zum einen enthält Pankreatin große Anteile nicht aktiver Bestandteile aus der Bauchspeicheldrüse. Daher ist die Aktivität relativ gering und es müssen große Mengen Pankreatin eingenommen werden, um eine wirksame Therapie zu erreichen. Je nach Formulierung muss hierbei eine große Anzahl an Kapseln oder Tabletten eingenommen werden; pro Mahlzeit können dies in Einzelfällen bis zu 8 - 15 Tabletten oder Kapseln sein. Dies ist unangenehm und führt häufig zu Compliance-Problemen. Zum anderen enthält das Pankreatin wechselnde Mengen an viralen und mikrobiellen Verunreinigungen, die aus der Bauchspeicheldrüse der Schweine stammen. Angesichts der Diskussion um die bovine spongiforme Enzephalopathie werden aus Säugetieren stammende biologische Verunreinigungen in Arzneimitteln von den Zulassungsbehörden immer kritischer gesehen. Zulassungen von Arzneimitteln, die aus tierischem Gewebe hergestellt werden, sind daher zunehmend problematisch. Noch problematischer ist die Verabreichung von Pankreatin bei Säuglingen und Kleinkindern. Diese können in der Regel keine Kapseln schlucken. Daher werden die Kapseln, die beispielsweise Pankreasenzymzubereitungen enthalten, geöffnet und die darin enthaltenen festen, magensaftresistent überzogenen, multipartikulären Einheiten auf oder in der Mahlzeit verteilt. Bei einer solchen peroralen Einnahme kann durch Kauen die Integrität des funktionell-magensaftresistenten Überzugs zerstört werden und die Enzyme an falscher Stelle, insbesondere vor der Magenpassage, freigesetzt, denaturiert und damit unwirksam gemacht werden. Außerdem kann Pankreatin nicht in flüssiger Form verabreicht werden, was ebenfalls für die Verwendung in der Pädiatrie ein Hindernis darstellt.

Zudem sind die in Pankreatin-Produkten stets vorliegenden Proteasen und Amylasen bei einigen Behandlungen unerwünscht: Der Amylase-Gehalt ist bei Kindern mit Mukoviszidose unerwünscht und Proteasen sind bei Patienten mit akuter Pankreatitis oder aktiven Phasen chronischer Pankreatitits kontraindiziert (siehe US 5 645 832). Deshalb wäre die Verfügbarkeit einer Lipase als einzelnes Protein vorteilhaft.

Die Verwendung von anderen Lipasen als der Pankreaslipase wurde vielfach vorgeschlagen. Insbesondere mikrobiologisch hergestellte Lipasen wurden für diesen Zweck offenbart. Die DE 1 642 654 A1 offenbart die Herstellung einer Lipase durch Fermentierung der Pilzart Rhizopus arrhizus und Gewinnung der Lipase aus der Mutterlauge. Die Verwendung der Lipase als Arzneistoff zur Behandlung von Pankreasinsuffizienzen und ähnlichen Krankheiten wird ebenfalls vorgeschlagen. Die EP 387 945 A beschreibt die Verwendung derselben Lipase in Kombination mit Pankreatin zur Behandlung von Pankreasinsuffizienzen. In der US 5,489,530 und der US 5,645,832 werden bakterielle Lipasen offenbart und deren Verwendung als Arzneimittel zur Behandlung von Pankreasinsuffizienzen erwogen.

Die US 2006/121017 A1 offenbart Zusammensetzungen, die bakterielle Lipase, Protease und Amylase enthalten. Die Lipase kann aus der Gattung *Pseudomonas* oder *Burkholderia* oder aus der Art *Burkholderia cepacia* stammen. Die WO 2010/025126 A1 offenbart schnell zerfallende Tabletten mit denselben Inhaltsstoffen, die zusätzlich einen Brausesatz enthalten. Die Tabletten lösen sich in der Mundhöhle schnell auf. Die Stabilisierung der Lipasen mittels Kristallisierung oder Quervernetzung ist vorgesehen. Die Verwendung von quervernetzten Lipase-Kristallen von Lipasen aus der Gattung Burkholderia in Mischungen mit einer bestimmten Protease und einer bestimmten Amylase wird offenbart.

Die WO 2010/085975 A1 offenbart schließlich flüssige Zubereitungsformen der Burlulipase für die Behandlung von Verdauungsstörungen, insbesondere von Pankreatitis und Mukoviszidose. Die Lipase wird aus der Gattung Burkholderia, der Gattung Pseudomonas oder aus der Art *Burkholderia plantarii* durch Fermentation und Gewinnung der Lipasen aus dem Kulturüberstand gewonnen.

Burlulipase hat jedoch Nachteile, die Ihre praktische Verwendung in Arzneimitteln bisher verhindert haben. Sie ist thermisch instabil in flüssigen Lösungen und solche Lösungen können daher nicht bei Raumtemperatur gelagert werden, was nachteilig ist. Die Herstellung von Tabletten und Granulaten geht in der Regel mit einen erheblichen Verlust der Aktivität einher (unveröffentlichte Daten). Bei der Tablettierung werden für gewöhnlich 20 bis 70 % der Burlulipase inaktiviert. Dies führt nicht nur zu einer Herabsetzung der Lipaseaktivität, sondern auch zu einer nicht akzeptablen Abweichung des Wirkstoffgehalts bei verschiedenen Chargen.

Hingegen gelingt es mit Burlulipase im Gegensatz zu Pankreatin, Arzneimittel mit nur einem einzelnen Verdauungsenzym bereitzustellen. Ferner ist die spezifische Aktivität von Burlulipase sehr hoch, so dass nur geringe Substanzmengen verabreicht werden müssen. Der pH-Bereich für die Stabilität und die Aktivität der Burlulipase liegt zwischen pH 4 und pH 9 und überwindet daher die Einschränkungen der Stabilität und Aktivität von Schweine-Lipase bzw. Schweine-Pankreatin, die im Magen deaktiviert werden. Für begrenzte Zeiträume übersteht Burlulipase auch Belastungen bei pH-Werten noch deutlich unterhalb von pH 4. Dies bedeutet, dass die lipolytische Wirkung der Bakterien-Lipasen mit größerer Wirkung im Magen-Darm-Trakt angewendet werden kann, als die für die Therapie von Verdauungsstörungen auf dem Markt geläufigen Produkte. Dass Burlulipase beim pH-Wert des Magensafts im Gegensatz zum Pankreatin über eine gewisse Zeit weitgehend stabil ist, lässt sie für die Herstellung von peroral verabreichten Zubereitungsformen geeignet erscheinen.

Die WO 2010/085 975 und die DE 10 2009 06 5694 A1 offenbaren pharmazeutische Präparate zur Behandlung von Pankreasinsuffizienz, beispielsweise Mukoviszidose oder anderer Pankreaserkrankungen. Insbesondere werden flüssige pharmazeutische Präparate von Verdauungsenzymen, die keinen Überzug haben und in der aggressiven Umgebung von Magen/Zwölffingerdarm aktiv sind, offenbart. Außerdem sind die vorgeschlagenen flüssigen pharmazeutischen Präparate stabil und können bei hohen Temperaturen gelagert werden.

Die WO 2006/044 529 A1 bezieht sich auf eine Zusammensetzung zur Behandlung von Zuständen einschließlich Pankreasinsuffizienz. Die Zusammensetzungen der vorliegenden Erfindung umfassen Lipase, Protease und Amylase in einem bestimmten Verhältnis, das vorteilhafte Ergebnisse z.B. bei Patienten, die von Pankreasinsuffizienz betroffen sind, bewirkt. Der Vorschlag bezieht sich auch auf Verfahren, die solche Zusammensetzungen zur Behandlung von Pankreasinsuffizienz verwenden.

### Aufgabe der vorliegenden Erfindung

Es ist die Aufgabe der vorliegenden Erfindung, ein Arzneimittel enthaltend Burlulipase zur Verfügung zu stellen. Dieses Arzneimittel soll die vorstehend genannten Probleme der Burlulipase lösen oder lindern. Insbesondere soll das Arzneimittel ohne großen Verlust der Aktivität herstellbar sein. Es ist ferner eine Aufgabe der vorliegenden Erfindung, ein Arzneimittel zur Verfügung zu stellen, dass ohne großen Verlust der Aktivität lagerbar ist. Zudem soll das Arzneimittel in genau festgelegten Dosen herstellbar sein. Ferner soll ein Verfahren bereitgestellt werden, das es erlaubt ein solches Arzneimittel herzustellen, ohne dass die Aktivität der eingesetzten Burlulipase während des Herstellungsprozesses stark abnimmt. Zusätzlich soll das Arzneimittel die Herstellung von flüssigen Zubereitungsformen der Burlulipase ermöglichen, bei denen die Aktivität der Burlulipase gegenüber der Aktivität der ursprünglich zur Herstellung des Arzneimittels eingesetzten Burlulipase nur geringfügig oder gar nicht herabgesetzt ist. Das Arzneimittel soll die Verabreichung der Burlulipase in flüssigen Zubereitungsformen ermöglichen. Es ist daher auch eine Aufgabe der vorliegenden Erfindung eine flüssige pharmazeutische Zusammensetzung zur Verfügung zu stellen, die in der Pädiatrie Verwendung finden kann und/oder die zur Behandlung der Mukoviszidose und/oder zur Sondengabe bei künstlicher Ernährung geeignet ist.

### Beschreibung der Erfindung

### Schmelztabletten

Ein Aspekt der vorliegenden Erfindung ist eine Schmelztablette die dadurch gekennzeichnet ist, dass sie Burlulipase enthält. Burlulipase (Internationaler Freiname; INN) ist die Lipase der Bakterienart *Burkholderia plantarii.* Burlulipase ist eine Triacylglycerolacylhydrolase (EC 3.1.1.3), die eine mit den durch *Burkholderia plantarii* und *Burkholderia glumae* produzierten Lipasen übereinstimmende Aminosäuresequenz aufweist. Die Burlulipase wird hergestellt durch einen klassischen Fermentationsprozess, in dem *Burkholderia plantarii,* ein nicht-rekombinantes, gramnegatives Bakterium als Produktionsstamm eingesetzt wird. Reine Burlulipase kann eine spezifische Aktivität von mehr als 3.500 U/mg (Tributyrin-Einheiten pro Milligramm Protein) aufweisen. Aufgrund dieser hohen lipolytischen Aktivität ist Burlulipase zur Unterstützung der Verdauungsleistung bei gesunden und kranken Menschen besonders geeignet. Burlulipase kann in sehr hohen Konzentrationen mit hoher Aktivität erhalten werden, so dass nur geringe Substanzmengen (d. h. eine geringe Masse bzw. ein geringes Volumen an Lösung) verabreicht werden müssen. Die Eignung von flüssigen Zubereitungen der Burlulipase zur Behandlung von Verdauungsstörungen ist aus der WO 2010/085975 A1 bekannt.

Die Herstellung fester Zubereitungsformen der Burlulipase geht in der Regel mit einem großen Verlust der Aktivität der Burlulipase einher. Typischerweise verbleiben nur etwa 60 % der ursprünglichen Aktivität der eingesetzten Burlulipase in den Tabletten. Das Ausmaß des Aktivitätsverlustes hängt auch von den Verfahrensbedingungen ab. Gleichzeitig scheint Burlulipase empfindlich gegen einige der in Tabletten eingesetzten Hilfsstoffe zu sein. Überraschenderweise konnte jedoch hier gezeigt werden, dass es möglich ist, Schmelztabletten enthaltend Burlulipase herzustellen, deren Herstellung praktisch ohne Verlust der Aktivität der Burlulipase möglich ist. Die Schmelztabletten können leicht hergestellt werden und erlauben die Lagerung bei Raumtemperatur über einen für Arzneimittel üblichen Zeitraum. Auch bei Lagerung über mehrere Jahre sinkt die Aktivität der darin enthaltenen Burlulipase lediglich in einem pharmazeutisch akzeptablen Ausmaß.

Zusätzlich erlauben die erfindungsgemäßen Schmelztabletten die einfache und schnelle Herstellung von Lösungen, Emulsionen oder Suspensionen durch einfaches Zusammengeben der Schmelztabletten mit einer Flüssigkeit wie zum Beispiel Wasser, Getränken oder dergleichen, gegebenenfalls unter Rühren. Schmelztabletten ermöglichen es daher, flüssige Arzneimittel zur Verfügung zu stellen, ohne dass die Zubereitungsform bei Lagerung gekühlt werden muss. Flüssige Zubereitungsformen der Burlulipase sind hingegen thermisch instabil und können nur in Form von gekühlten Lösungen in den Handel gebracht werden, mit allen Problemen die eine ununterbrochene Kühlkette mit sich bringt. Schmelztabletten können hier erfolgreich eingesetzt werden um die Lagerung flüssiger Zubereitungsformen zu umgehen. Auch die Verpackung ist einfacher als bei Flüssigkeiten.

Gemäß dem Europäischen Arzneibuch, 8. Ausgabe (*Ph. Eur*.) Band 1 - allgemeiner Teil, Monographiegruppen, wird für Schmelztabletten eine Zerfallszeit von maximal drei Minuten (in Wasser) verlangt. Die hier beschriebenen Schmelztabletten lösen sich in Wasser bei 20 °C in weniger als 2 Minuten auf. Unter Rühren losen sie sich jedoch in der Regel innerhalb von wenigen Sekunden auf, typischer weise in weniger als 10 Sekunden, meist in 2 bis 5 Sekunden.

Es ist bevorzugt, dass die Schmelztablette, wie hierin beschrieben, ein Lyophilisat enthält. Besonders bevorzugt enthält sie ein Lyophilisat einer Burlulipase-haltigen Zusammensetzung dar. Solche Lyophilisate können aus Lösungen, Emulsionen oder Suspensionen von Burlulipase z.B. durch Gefriertrocknung hergestellt werden. Alternative Herstellungsverfahren sind beispielsweise Sprühtrocknung, Vakuumtrocknung oder Sprühgefriertrocknung. Bei der Gefriertrocknung und Sprühgefriertrocknung kann die Herstellung schonend bei tiefen Temperaturen erfolgen. Bevorzugt werden die Lyophilisate aus Lösungen hergestellt. Lösungen ergeben sehr homogene und hochporöse Lyophilisate, die sich schnell in Flüssigkeiten auflösen. Ganz besonders bevorzugt ist eine erfindungsgemäße Schmelztablette die ein Lyophilisat einer wässrigen Lösung der Burlulipase enthält. Solche Lösungen ergeben Lyophilisate, in denen der Wirkstoff sehr homogen verteilt ist. Ferner liegt die Temperatur bei der Gefriertrocknung bevorzugt bei 0 °C oder weniger. Bei solchen Temperaturen ist die Burlulipase stabil. Ganz besonders bevorzugt ist es, wenn die erfindungsgemäße Schmelztablette ein Lyophilisat einer wässrigen Lösung der Burlulipase enthält, die bei einer Temperatur von 0 °C oder weniger gefriergetrocknet wurde. Am meisten bevorzugt werden die Lösungen, Emulsionen oder Suspensionen von Burlulipase mittels flüssiger Luft gekühlt, bevor sie gefriergetrocknet werden. Eine schnelle Abkühlung führt zu einer homogenen gefrorenen Masse. Bei der Gefriertrocknung werden weniger Hilfsstoffe eingesetzt als beim Pressen einer klassischen Tablette und die notwendigen Hilfsstoffe wirken hier nicht bzw. kaum inaktivierend auf Burlulipase. Am meisten bevorzugt ist die Verwendung von Lösungen der Burlulipase zur Herstellung des Lyophilisats, wie sie nach Expression der Burlulipase und Aufarbeitung des Kulturüberstands entstehen. Diese Lösungen können dann mit Hilfsstoffen versetzt und lyophilisiert werden. Besonders bevorzugt besteht die Schmelztablette aus einem Lyophilisat wie hierin beschrieben.

Bei der Herstellung der erfindungsgemäßen Schmelztabletten wird die Burlulipase überraschender Weise nicht oder kaum inaktiviert. Ferner sind die Schmelztabletten überraschender Weise bei Raumtemperatur lagerstabil. Auf eine künstliche Stabilisierung der Burlulipase kann daher verzichtet werden kann. Die Verwendung von kristalliner Burlulipase wird unnötig. Auch eine Quervernetzung der Burlulipase ist nicht notwendig. Beides wird in der Literatur zur Stabilisierung von Lipasen vorgeschlagen (siehe z.B. WO 1010/025126, Absatz [033]). Das Kristallisieren ist jedoch ein aufwändiger, nicht leicht reproduzierbarer Prozess und die Quervernetzung ist ebenfalls aufwändig und hat üblicherweise eine teilweise Inaktivierung der Burlulipase zur Folge. Die erfindungsgemäße Schmelztablette ist daher bevorzugt dadurch gekennzeichnet, dass die darin enthaltene Burlulipase chemisch nicht modifiziert ist. Sie ist ferner bevorzugt dadurch gekennzeichnet, dass die darin enthaltene Burlulipase nicht in kristalliner Form vorliegt, also amorph ist. Besonders bevorzugt ist die Burlulipase chemisch nicht modifiziert und liegt in amorpher Form vor. Die erfindungsgemäße Schmelztablette enthält bevorzugt 0,01 Gew.-% bis 90 Gew.-% Burlulipase, besonders bevorzugt 0,1 Gew.-% bis 40 Gew.-%, ganz besonders bevorzugt 1 Gew.-% bis 30 Gew.-% und am meisten bevorzugt 2 Gew.-% bis 20 Gew.-%. So weit nicht anders angegeben, beziehen sich alle prozentualen Mengenangaben in diesem Text auf die Gesamtmasse der Schmelztablette ohne den Restwassergehalt, wie nach Karl Fischer bestimmt.

Da Burlulipase durch Druckeinwirkung inaktiviert werden kann, sollten erhöhte Drücke so weit wie möglich vermieden werden. Bevorzugt sind daher erfindungsgemäße Herstellungsverfahren für die erfindungsgemäßen Schmelztabletten, bei denen auf Pressvorgänge verzichtet wird. Besonders bevorzugt sind erfindungsgemäße Herstellungsverfahren für die erfindungsgemäßen Schmelztabletten bei denen auf jegliche mechanische Druckeinwirkung zur Verdichtung oder Verfestigung der Schmelztabletten verzichtet wird.

Die erfindungsgemäßen Schmelztabletten enthalten außer der Burlulipase noch Hilfsstoffe. Hilfsstoffe im Sinne dieser Anmeldung sind alle Hilfsmittel die üblicherweise in pharmazeutischen Zusammensetzungen verwendet werden. Wirkstoffe, insbesondere Enzyme, sind keine Hilfsstoffe im Sinne dieser Anmeldung. Hilfsstoffe sind zum Beispiel im "Handbook of Pharmaceutical Excipients" der "American Pharmaceutical Association" aufgeführt. Für die erfindungsgemäßen Schmelztabletten besonders geeignete Hilfsstoffe sind zum Beispiel in Absatz [0019] der EP 1 804 764 B1 aufgeführt. Die erfindungsgemäße Schmelztablette enthält bevorzugt 10 Gew.-% bis 99,99 Gew.-% Hilfsstoffe, besonders bevorzugt 60 Gew.-% bis 99,9 Gew.-%, ganz besonders bevorzugt 70 Gew.-% bis 99 Gew.-% und am meisten bevorzugt 80 Gew.-% bis 98 Gew.-%.

Bevorzugt enthält die erfindungsgemäße Schmelztablette zusätzlich zu der Burlulipase wenigstens ein Bindemittel. Prinzipiell sind alle bekannten Bindemittel geeignet. Bevorzugt ist das Bindemittel jedoch ausgesucht aus der Gruppe bestehend aus Gelatine, hydrolysierte Gelatine, Polyvinylpyrrolidon z. B. Kollidon®), Celluloseether und vorgelierte Stärke. Besonders bevorzugt ist Gelatine und am meisten bevorzugt ist Fischgelatine. Insbesondere kann es sich um unveränderte natürliche Fischgelatine, um nichtgelierende Fischgelatine, um hydrolysierte Fischgelatine oder um sprühgetrocknete Fischgelatine handeln. Die Bindemittel können in den vorstehend für die Hilfsstoffe angegebenen Mengen vorhanden sein. Bevorzugt enthält die erfindungsgemäße Schmelztablette wenigstens einen Hilfsstoff, der ausgesucht ist aus der Gruppe bestehend aus Bindemitteln und strukturbildenden Hilfsstoffen.
Prinzipiell sind alle bekannten Füllstoffe und strukturbildenden Hilfsstoffe geeignet. Bevorzugt sind diese ausgewählt aus der Gruppe bestehend aus Zuckeralkoholen, Zuckern, Cellulosepulver, Calciumsulfat und mikrokristalline Cellulose. Bevorzugt sind Zuckeralkohole und Zucker und am meisten bevorzugt ist Mannitol. Die strukturbildenden Hilfsstoffe können in den vorstehend für die Hilfsstoffe angegebenen Mengen vorhanden sein.

Bevorzugt enthält die erfindungsgemäße Schmelztablette 0,01 Gew.-% - 90 Gew.-% Burlulipase, 5 Gew.-% - 90 Gew.-% Bindemittel und 5 Gew.-% - 90 Gew.-% strukturbildende Hilfsstoffe. Besonders bevorzugt enthält die erfindungsgemäße Schmelztablette 0,1 Gew.-% - 40 Gew.-% Burlulipase, 20 Gew.-% - 80 Gew.-% Bindemittel und 20 Gew.-% - 80 Gew.-% strukturbildende Hilfsstoffe. Ganz besonders bevorzugt enthält die erfindungsgemäße Schmelztablette 1 Gew.-% - 30 Gew.-% Burlulipase, 25 Gew.-% - 55 Gew.-% Bindemittel und 20 Gew.-% - 50 Gew.-% strukturbildende Hilfsstoffe. Am meisten bevorzugt enthält die erfindungsgemäße Schmelztablette 2 Gew.-% - 20 Gew.-% Burlulipase, 30 Gew.-% - 50 Gew.-% Bindemittel und 25 Gew.-% - 45 Gew.-% strukturbildende Hilfsstoffe.

Weiter kann die erfindungsgemäße Schmelztablette Hilfsstoffe enthalten, die ausgesucht sind aus der Gruppe bestehend aus Konservierungsmittel, Stabilisatoren, Netzmittel, Emulgatoren, Lösungshilfen, Salze zur Regulierung des osmotischen Drucks, Sprengmittel, Brausesätze und pH-Puffer. Bevorzugt enthält oder besteht die erfindungsgemäße Schmelztablette aus Burlulipase, Bindemittel, strukturbildende Hilfsstoffe, Emulgatoren und gegebenenfalls eine Säure oder Base zur Einstellung des pH-Wertes. Besonders bevorzugt enthält oder besteht die erfindungsgemäße Schmelztablette aus Burlulipase, Bindemittel, strukturbildende Hilfsstoffe und gegebenenfalls eine Säure oder Base zur Einstellung des pH-Wertes. Am meisten bevorzugt enthält oder besteht die erfindungsgemäße Schmelztablette aus Burlulipase, Fischgelatine, Mannitol und Natriumhydroxid. Ebenfalls besonders bevorzugt sind die erfindungsgemäßen Schmelztabletten aus Burlulipase, Fischgelatine, Mannitol und Zitronensäure. Ebenfalls besonders bevorzug sind die erfindungsgemäßen Schmelztabletten aus Burlulipase, Fischgelatine, Mannitol und Poloxamer. Ebenfalls besonders bevorzug sind die erfindungsgemäßen Schmelztablette aus Burlulipase, Fischgelatine, Mannitol und Polysorbat.

Zusätzlich zu allen anderen Inhaltsstoffen kann die hierin offenbarte erfindungsgemäße Schmelztablette auch Restmengen an Wasser enthalten. Bevorzugt ist die Restwassermenge in der Schmelztablette 10 Gew.-% oder weniger, bevorzugt 7 Gew.-% oder weniger und am meisten bevorzugt 5 Gew.-% oder weniger. Es ist ferner bevorzugt, dass die Restwassermenge im Bereich von 0,1 bis 10 Gew.-%, bevorzugt im Bereich von 0,5 bis 6 Gew.-% und besonders bevorzugt im Bereich von 1 bis 5 Gew.-% liegt. Die Restwassermengen sind jeweils bezogen auf die gesamte Masse der Schmelztablette.

Bevorzugt sind ebenfalls erfindungsgemäße Schmelztabletten, die Burlulipase, Fischgelatine, Mannitol, Natriumhydroxid und Restmengen von Wasser enthalten. Ganz besonders bevorzugt, sind erfindungsgemäße Schmelztabletten, die aus Burlulipase, Fischgelatine, Mannitol, Natriumhydroxid und Restmengen von Wasser bestehen. Insofern eine Säure oder Base zur Einstellung des pH-Wertes verwendet wird, ist mit der Angabe, dass die Schmelztablette diese Säure oder Base enthält, auch der Zustand gemeint, bei dem ein Teil der Säure oder Base durch andere Bestandteile der Schmelztablette neutralisiert ist und somit in Form ihrer Salze vorliegt.

Die bevorzugten Hilfsmittel beeinträchtigen die Stabilität der Burlulipase während der Lagerung der Schmelztabletten nicht oder nicht wesentlich. Gleiches gilt für die flüssigen Zusammensetzungen, die gefriergetrocknet werden, während der Gefriertrocknung und für die flüssigen Zusammensetzungen, wenn sie für die Gefriertrocknung vorbereitet werden. Diese können jedoch nicht über längere Zeit bei mehr als etwa 10 °C aufbewahrt werden und müssen gegebenenfalls gekühlt werden, da sie sonst teilweise inaktiviert werden. Dies ist aber in aller Regel nicht dem Einfluss der angegebenen Hilfsstoffe zuzuschreiben. Es handelt sich vielmehr um eine Eigenschaft der Burlulipase.

Weiter können die erfindungsgemäßen Schmelztabletten Stabilisatoren enthalten. Dabei kann es sich um solche Stabilisatoren handeln, die die Burlulipase in den Schmelztabletten selbst stabilisieren. Ferner kann es sich um solche Stabilisatoren handeln, die die Burlulipase in den flüssigen Zusammensetzungen stabilisieren, die verwendet werden, um die Schmelztabletten durch Gefriertrocknung herzustellen. Auch kann es sich um solche Stabilisatoren handeln, die die Burlulipase in den flüssigen Arzneimitteln stabilisieren, die aus den Schmelztabletten durch Auflösen, Emulgieren oder Suspendieren hergestellt werden (siehe weiter unten). Schließlich kann es sich um solche Stabilisatoren handeln, die die Burlulipase im Körper nach der Einnahme und insbesondere im Magen-Darm-Trakt stabilisieren. Bevorzugt sind die Stabilisatoren ausgesucht aus der Gruppe bestehend aus Salzen, organischen Säuren, Aminosäuren, Detergentien, Zuckern, Ölen, Viskositätsregulierungsmitteln. Insbesondere bevorzugt ist eine erfindungsgemäße Schmelztablette, die Calciumchlorid als Stabilisator enthält.

Die erfindungsgemäße Schmelztablette kann neben der Burlulipase weitere Wirkstoffe enthalten.

Gegen manche Brausesätze und Sprengmittel ist Burlulipase empfindlich. Als Folge hochenergetischer Grenzflächenphänomene an der Grenzfläche der aus einem Brausesatz resultierenden Gasblasen kann die Burlulipase inaktiviert werden. Bevorzugt ist daher eine Schmelztablette wie hierin beschrieben, die keinen Brausesatz enthält. Ebenfalls bevorzugt ist eine Schmelztablette, die keine Sprengmittel enthält. Am meisten bevorzugt ist eine Schmelztablette, die weder Brausesatz noch Sprengmittel enthält.

Die Schmelztablette wie hierin beschrieben enthält bevorzugt 0,1 bis 20 mg Burlulipase-Protein, besonders bevorzugt enthält sie 0,5 bis 10 mg und ganz besonders bevorzugt 1 bis 5 mg. Dabei richtet sich die Menge der Burlulipase natürlich nach deren Aktivität und der notwendigen Dosierung für eine bestimmte Behandlung. Es können daher auch Mengen von weniger als 0,1 mg oder Mengen von mehr als 20 mg Burlulipase-Protein eingesetzt werden.

Die erfindungsgemäße Schmelztablette wie hierin beschrieben kann neben der Burlulipase auch weitere Enzyme enthalten, insbesondere weitere Verdauungsenzyme. Als Verdauungsenzyme kommen insbesondere Enzyme ausgewählt aus der Gruppe bestehend aus Proteasen und Amylasen in Betracht. Auch Schmelztabletten die sowohl Protease als auch Amylase enthalten sind Teil der vorliegenden Erfindung. Bevorzugt sind jedoch Schmelztabletten die keine weiteren Enzyme enthalten. Solche Arzneiformen sind zur Behandlung von Krankheiten geeignet, bei denen die Anwesenheit von anderen Enzymen, wie Proteasen und Amylasen, unerwünscht ist. Zum Beispiel sind Proteasen bei akuter Pankreatitis oder aktiven Phasen chronischer Pankreatitis kontraindiziert. Amylasen sind bei Mukoviszidose besonders nachteilig.

### Flüssige pharmazeutische Zusammensetzung hergestellt aus Schmelztabletten

Ein weiterer Aspekt der vorliegenden Erfindung ist eine flüssige pharmazeutische Zusammensetzung umfassend Burlulipase, dadurch gekennzeichnet, dass sie durch Einbringen einer erfindungsgemäßen Schmelztablette, wie sie hierin beschrieben ist, in eine Flüssigkeit hergestellt wird. Die Schmelztabletten werden also gemäß derselben Methode wie bei der Verwendung von Alka-Seltzer® hergestellt und eingenommen. Eine solche flüssige pharmazeutische Zusammensetzung hat den Vorteil, dass sie leicht verabreicht werden kann, auch und insbesondere an Kinder. Die erfindungsgemäße flüssige pharmazeutische Zusammensetzung bietet die Möglichkeit einer bequemen Dosierung des Medikaments, der homogenen Verteilung der Burlulipase in der Nahrung und die Möglichkeit der Anwendung bei künstlicher Ernährung (Sondengabe). Die erfindungsgemäße flüssige pharmazeutische Zusammensetzung wird bevorzugt durch Einbringen einer erfindungsgemäßen Schmelztablette in ein Getränk hergestellt. Das Getränk kann zum Beispiel Wasser, Fruchtsaft, wie Orangensaft, Milch oder dergleichen sein. Bei der erfindungsgemäßen flüssigen pharmazeutischen Zusammensetzung kann es sich um eine Lösung, eine Suspension oder um eine Emulsion handeln. Bevorzugt ist eine Lösung. Am meisten bevorzugt sind wässrige Lösungen und darunter wiederum bevorzugt sind Lösungen in Trinkwasser.

Burlulipase liegt in der flüssigen pharmazeutischen Zusammensetzung bevorzugt in einer Konzentration von 0,0002 mg/ml bis 50 mg/ml, bevorzugt in einer Konzentration von 0,002 mg/ml bis 5 mg/ml und besonders bevorzugt in einer Konzentration von 0,001 mg/ml bis 2 mg/ml vor. Im Falle der Burlulipase sind diese Angaben bezogen auf das Burlulipase-Protein. Burlulipase liegt in der Regel vergesellschaftet mit Zuckern (z. B. 6-Desoxy-Talan) vor, die bei der Bestimmung des Gehaltes an Burlulipase jedoch nicht beachtet werden sollen. Dies gilt für alle Mengenangaben in Bezug auf Burlulipase in diesem Text.

Die erfindungsgemäßen Schmelztabletten können natürlich auch als solche peroral verabreicht werden und lösen sich dann innerhalb kürzester Zeit im Mundraum auf. Bevorzugt ist aber die Verabreichung als erfindungsgemäße flüssige pharmazeutische Zusammensetzung. Die erfindungsgemäßen Schmelztabletten ermöglichen erst die Bereitstellung von flüssigen pharmazeutischen Zusammensetzungen, die Burlulipase enthalten, da sie drei Probleme lösen, die bisher eine breite Verwendung der Burlulipase als Arzneimittel behindert haben. Sie sind leicht und ohne große Verluste der Aktivität der Burlulipase herstellbar. Sie können über lange Zeiträume ohne großen Verlust der Aktivität der Burlulipase aufbewahrt werden und sie können mit einfachen Mitteln direkt vor der Einnahme innerhalb von Sekunden in flüssige Arzneiformen überführt werden. Auch dies geschieht nahezu ohne Aktivitätsverlust.

Die erfindungsgemäße flüssige pharmazeutische Zusammensetzung ist bevorzugt ein Arzneimittel zur Vorbeugung und/oder Behandlung von Verdauungsproblemen oder zur Vorbeugung und/oder Behandlung von Erkrankungen, bei denen Verdauungsprobleme eine Rolle spielen. Bevorzugt wird mit dem Trinken des Getränks begonnen, nachdem mit der Mahlzeit begonnen wurde, bevorzugt nachdem etwa ¼ der Mahlzeit verzehrt wurde.

### Verfahren zur Herstellung der Schmelztabletten

Ein weiterer Aspekt der vorliegenden Erfindung ist ein Verfahren zur Herstellung einer erfindungsgemäßen Schmelztablette, wie sie hierin beschrieben ist, umfassend die Schritte:
- Bereitstellen einer wässrigen Lösung, Emulsion oder Suspension enthaltend die Burlulipase und Hilfsstoffe,
- Einfüllen der wässrigen Lösung, Emulsion oder Suspension in eine Form,
- Gefrieren der wässrigen Lösung, Emulsion oder Suspension in der Form,
- Gefriertrocknen der wässrigen Lösung, Emulsion oder Suspension in der Form.

Die Wiederfindungsrate der Aktivität der Burlulipase in der Schmelztablette im Vergleich mit der zur Herstellung des Lyophilisats verwendeten Lösung, Emulsion oder Suspension beträgt üblicherweise mehr als 90 %. Bevorzugt ist das vorstehend genannte Verfahren, bei dem wenigstens 90 %, besonders bevorzugt 95 % und am meisten bevorzugt 98 % der Aktivität der Burlulipase in der wässrigen Lösung, Emulsion oder Suspension die zur Herstellung der Schmelztablette verwendet wurde in der Schmelztablette wiedergefunden werden. Bevorzugt wird eine Lösung verwendet.

Für die Durchführung dieses Verfahrens sind alle hierin als für die Herstellung der erfindungsgemäßen Schmelztabletten beschriebenen Lösungen, Emulsionen oder Suspensionen geeignet. Ferner können diese Lösungen, Emulsionen oder Suspensionen alle für die Schmelztablette beschriebenen Inhaltsstoffe, insbesondere Hilfsstoffe, enthalten. Ferner können für das Gefrieren und das Gefriertrocknen alle hierin beschriebenen Verfahrensschritte und Verfahrensbedingungen ohne Einschränkung Anwendung finden. Als Form wird bevorzugt ein Blister verwendet, der für die Gefriertrocknung vorgesehen ist. Solche Blister sind zum Beispiel in der US 2002/112449 A beschrieben.

### Arzneimittel

Ein weiterer Aspekt der vorliegenden Erfindung ist ein Arzneimittel zur zur Anwendung in der Vorbeugung oder Behandlung von Lipaseinsuffizienz umfassend eine erfindungsgemäße Schmelztablette oder ein erfindungsgemäßes flüssiges Arzneimittel. Bevorzugt handelt es sich um ein Arzneimittel zur Vorbeugung oder Behandlung von Verdauungsproblemen bei Erwachsenen, Kleinkindern und Säuglingen. Bevorzugt handelt es sich um ein Arzneimittel zur Behandlung von Kleinkindern und Säuglingen. Ebenfalls bevorzugt handelt es sich um ein Arzneimittel zur Behandlung von Verdauungsstörungen, insbesondere als Folge von Pankreatitis, und Verdauungsstörungen, die bei Mukoviszidose auftreten. Ganz besonders bevorzugt handelt es sich um ein Arzneimittel zur Vorbeugung und/oder Behandlung von Pankreaserkrankungen, wie Pankreatitis, insbesondere exokriner Pankreasinsuffizienz und Pankreaserkrankungen im Zusammenhang mit Mukoviszidose, wie Pankreatitis, insbesondere exokriner Pankreasinsuffizienz.

### Beispiele

### Reagenzien/Hilfsstoffe:

Avicel® PH-101: Mikrokristalline Cellulose der Firma FMC;
Emcompress: Calciumhydrogenphosphat-Dihydrat der Firma JRS Pharma; Rosenberg, Deutschland;
Kollidon® CL: Polyvinylpyrrolidon der Firma BASF SE, Ludwigshafen, Deutschland;
Aerosil®: Pyrogene Kieselsäure der Firma Evonik Industries AG, Essen, Deutschland.

### Analyseverfahren:

Die analytische Bestimmung der lipolytischen Aktivität erfolgt soweit nicht anders angegeben durch den sog. Tributyrin-Assay gemäß Erlanson, Ch. & Borgström, B.: "Tributyrine as a Substrate for Determination of Lipase Activity of Pancreatic Juice and Small Intestinal Content"; Scand. J. Gastroent. 5, 293-295 (1970). Die Angabe der durch den sog. Tributyrin-Assay ermittelten lipolytischen Aktivität erfolgt synonym in TBU-Einheiten (TBU-E., z. T. verkürzt zu TBU) bzw. TBU-Units (TBU-U., z. T. verkürzt zu TBU), wobei die Schreibweisen (mit/ohne Abkürzungspunkte, mit/ohne Bindestrich sowie mit/ohne Leerzeichen) in der wissenschaftlichen Literatur z. T. stark variieren. Dabei entspricht eine enzymatische Aktivität von 1 Unit (1 Enzymeinheit) einem Stoffumsatz von 1 µmol Substrat pro Minute.

Die angegebenen Werte sind normalisierte Werte, die auf dem Proteingehalt der Burlulipase bezogen sind. Der Restwassergehalt wird nach Karl Fischer bestimmt. Gegebenenfalls wird hierbei Formamid als Lösungsvermittler verwendet.

Die analytische Bestimmung der Bruchfestigkeit erfolgt gemäß der diesbezüglichen Methode des Europäischen Arzneibuchs - Ph. Eur. "2.9.8 Bruchfestigkeit von Tabletten" - mit einem Bruchfestigkeitstester des Typs Schleuniger 6D.

### Beispiel 1: Herstellen einer Schmelztablette

### Herstellung einer Lösung für die Gefriertrocknung

26,11 Teile Wasser, 5 Teile Fischgelatine und 4 Teile Mannitol werden zusammengegeben und unter Rühren auf 60 °C ± 2 °C erwärmt. Es wird auf 8 °C ± 2 °C abgekühlt und der pH-Wert der so entstandenen Lösung wird durch Zugabe einer 3 Gew.-% Lösung von Natriumhydroxid in Wasser auf 7,75 ± 0,25 eingestellt. Hierzu werden 1,44 Teile der Natronlauge benötigt. Anschließend werden 63,45 Teile einer Burlulipase-Lösung hinzugegeben und es wird gut gemischt. Die Burlulipase-Lösung hat eine Konzentration der Burlulipase von 23,64 mg Protein/ml.

### Gefriertrocknen

Je 200 mg der so hergestellten Lösung werden in je eine Tasche eines Blisters eingebracht. Die Taschen haben die Form von Tabletten mit einem Durchmesser von 11,50 mm und einer Höhe von 2,50 mm. Dann wird der mit der Lösung gefüllte Blister innerhalb von 3 bis 4 Minuten auf -80 °C gekühlt. Das gefrorene Produkt wird anschließend bei 0 °C über eine Zeitdauer von etwa 9 Stunden bis zu einem Feuchtigkeitsgehalt von weniger als 5 Gew.-% getrocknet. Die so hergestellte Tablette enthält 3 mg Burlulipase-Protein. Nach Auflösen der Tablette in Wasser betrug die Wiederfindungsrate der Burlulipaseaktivität 98,7 % der Aktivität der ursprünglich eingesetzten Burlulipaselösung. Bei dieser Wiederfindungsrate kann im Wesentlichen davon ausgegangen werden, dass die Verluste nur durch Anhaften von Burlulipase an den verwendeten Geräten entsteht und die Burlulipase praktisch nicht zersetzt oder deaktiviert wird. Die Schmelztablette weist einen akzeptablen visuellen Eindruck auf. Sie löst sich in 100 ml Wasser bei 18,8 °C unter Rühren innerhalb von 10 Sekunden rückstandsfrei auf.

### Beispiel 2: Test der Lagerstabilität

Die hergestellten Blister der Schmelztabletten wurden über 3 Monate bei 40 °C und 75 % Luftfeuchte gelagert. Nach Auflösen der Tablette in Wasser betrug die Wiederfindungsrate der Burlulipaseaktivität 96,1 % der Aktivität der ursprünglich eingesetzten Burlulipaselösung. Eine Lagerung bei Raumtemperatur über 36 Monate ergab keine Änderung der Burlulipaseaktivität. Diese Werte legen nahe, dass die hergestellten Schmelztabletten genügend lagerstabil für die Verwendung als Arzneimittel sind.

### Beispiel 3: Herstellen eines flüssigen Arzneimittels

Die Tablette einer Tasche des Blisters wird in 100 ml Evian®-Wasser gegeben. Die Tablette löst sich unter Rühren innerhalb von 2 Sekunden rückstandsfrei auf.

### Beispiel 4: Vergleichsbeispiel

Herstellung einer Tablette enthaltend Burlulipase (gefriergetrocknetes Lyophilisat aus eigener Herstellung) durch klassisches Verpressen

Die analytische Bestimmung der lipolytischen Aktivität erfolgte in diesem Beispiel durch die diesbezügliche Methode des Weltapothekerverbands (Fédération Internationale Pharmaceutique, FIP) gemäß Demeester, J. et al.: "XI. Microbial Lipases (F.I.P.)"; Drugs and the pharmaceutical sciences: 84, Pharmaceutical enzymes, 379-382 (1997). Die Angabe der durch diese Methode ermittelten lipolytischen Aktivität erfolgt in FIP-Einheiten (FIP-E.) bzw. FIP-Units (FIP-U.), wobei die Schreibweisen (mit/ohne Abkürzungspunkte, mit/ohne Bindestrich sowie mit/ohne Leerzeichen) in der wissenschaftlichen Literatur z. T. stark variieren.

In einem Zoller-Freifallmischer wurden die Bestandteile 1) bis 6) für 10 min gemischt. Nach Zugabe von Komponente 7) wurde nochmals für 5 min endgemischt.

| | |
|---|---|
| 1) Burlulipase-Lyophilisat | 11,02 g |
| (Lipolytische Aktivität: 3660 FIP-E./mg) | |
| 2) Avicel® PH-101 | 38,77 g |
| 3) Emcompress® | 42,01 g |
| 4) Talkum Ph. Eur. | 4,67 g |
| 5) Kollidon® CL | 1,75 g |
| 6) Aerosil® | 0,78 g |
| 7) Magnesiumstearat Ph. Eur. | 1,00 g |
| Gesamtmasse: | 100,00 g |

Die so erhaltene pressfertige Masse wurde zu Tabletten mit einer Durchschnittsmasse von 135,6 mg verpresst. Hierzu wurde eine Exzenter-Tablettenpresse des Typs Korsch EK 0 (erhältlich von der KORSCH Aktiengesellschaft, Berlin, Deutschland) verwendet, bestückt mit einem 7,0 mm-Stempel (drageegewölbt). Der Pressdruck betrug 21 kN. Die Höhe der Tabletten betrug im Mittel 3,46 mm, die Bruchfestigkeit der Tabletten betrug im Mittel 157 N.

Das eingesetzte Burlulipase-Lyophilisat (Wirkstoff) weist eine spezifische lipolytische Aktivität von 3660 FIP-E./mg auf. Da 11,02 g eingesetzt werden, wird insgesamt eine Menge von Burlulipase mit einer lipolytischen Gesamtaktivität von ca. 40,33 Millionen FIP-E. eingesetzt. Da die Gesamtmasse der eingesetzten Bestandteile 100 g beträgt, ergibt sich somit rechnerisch eine spezifische Aktivität von 403,3 FIP-E./mg für die Gesamtmasse der Bestandteile der zur Verpressung vorgesehenen Mischung. Nach dem Mischen wird in der pressfertigen Masse eine spezifische lipolytische Aktivität von 335 FIP-E./mg gemessen. Damit tritt allein durch das Mischen ein Verlust der lipolytischen Aktivität von 16,9 % auf. Das Endprodukt, also die gepresste Tablette, weist nur noch eine spezifische lipolytische Aktivität von 244 FIP-E./mg auf. Damit tritt durch das Pressen ein weiterer Verlust der lipolytischen Aktivität von 27,2 % (verglichen mit der spezifischen lipolytischen Aktivität der pressfertigen Masse von 335 FIP-E./mg) ein. Der Gesamtverlust an lipolytischer Aktivität durch das Verarbeiten eines Lyophilisats der Burlulipase zu einer Tablette beträgt demnach 39,5 % (Reduktion von 403,3 FIP-E./mg auf 244 FIP-E./mg). Da Burlulipase bei der Herstellung üblicherweise zunächst als flüssige Lösung anfällt, fällt zusätzlich noch ein Aktivitätsverlust durch das Lyophilisieren an.

Damit beträgt die Wiederfindungsrate der Burlulipase-Aktivität bei der klassischen Herstellung von Tabletten durch Mischen und Verpressen lediglich 60,5 % der Aktivität des ursprünglich eingesetzten Burlulipase-Lyophilisats. Im Vergleich dazu beträgt die Wiederfindungsrate der Burlulipase-Aktivität bei der Herstellung der erfindungsgemäßen Schmelztablette 98,7 % der Aktivität der ursprünglich eingesetzten Burlulipase-Lösung.

## Patentansprüche

1. Schmelztablette, **dadurch gekennzeichnet, dass** sie Burlulipase enthält.

2. Schmelztablette gemäß Anspruch 1, **dadurch gekennzeichnet, dass** sie ein Lyophilisat enthält.

3. Schmelztablette gemäß einem der Ansprüche 1, oder 2, **dadurch gekennzeichnet, dass** sie ein Lyophilisat einer wässrigen Lösung der Burlulipase enthält.

4. Schmelztablette gemäß einem der Ansprüche 1 bis 3, **dadurch gekennzeichnet, dass** sie ein Lyophilisat einer wässrigen Lösung der Burlulipase enthält, die bei einer Temperatur von 0°C oder weniger gefriergetrocknet wurde.

5. Schmelztablette gemäß einem der Ansprüche 1 bis 4, **dadurch gekennzeichnet, dass** sie keinen Brausesatz und keine Sprengmittel enthält.

6. Schmelztablette gemäß einem der Ansprüche 1 bis 5, **dadurch gekennzeichnet, dass** sie wenigstens einen Hilfsstoff enthält, der ausgesucht ist aus der Gruppe bestehend aus Bindemitteln und strukturbildenden Hilfsstoffen.

7. Schmelztablette gemäß einem der Ansprüche 1 bis 6, **dadurch gekennzeichnet, dass** sie Burlulipase, Bindemittel, strukturbildende Hilfsstoffe und gegebenenfalls eine Säure oder Base zur Einstellung des pH-Wertes und/oder ein Tensid zur Verbesserung des Zerfalls bzw. des Auflösungsverhaltens umfasst.

8. Schmelztablette gemäß Anspruch 7, **dadurch gekennzeichnet, dass** sie Burlulipase, Fischgelatine, Mannitol und Natriumhydroxid enthält.

9. Schmelztablette gemäß einem der Ansprüche 1 bis 8, **dadurch gekennzeichnet, dass** sie 0,1 bis 20 mg Burlulipase-Protein enthält.

10. Schmelztablette gemäß einem der Ansprüche 1 bis 8, **dadurch gekennzeichnet, dass** sie in einem 200 µl-Blister 0,1 bis 20 mg, bevorzugt 0,5 bis 10 mg und ganz besonders bevorzugt 1 bis 5 mg Burlulipase-Protein enthält oder **dadurch gekennzeichnet, dass** sie in einem 1200 µl-Blister zwischen 5 und 25 mg Burlulipase-Protein enthält.

11. Flüssige pharmazeutische Zusammensetzung umfassend Burlulipase, **dadurch gekennzeichnet, dass** sie durch Einbringen einer Schmelztablette gemäß einem der Ansprüche 1 bis 10 in eine Flüssigkeit hergestellt wird, **dadurch gekennzeichnet, dass** die Schmelztablette wenigstens ein Bindemittel umfasst.

12. Arzneimittel zur Anwendung in der Vorbeugung und/oder Behandlung von Lipasemangelerkrankungen umfassend eine Schmelztablette nach einem der Ansprüche 1 bis 10 oder eine flüssige pharmazeutische Zusammensetzung nach Anspruch 11.

13. Arzneimittel zur Anwendung in der Vorbeugung und/oder Behandlung von Lipasemangelerkrankungen gemäß Anspruch 12,
**dadurch gekennzeichnet,**
**dass** es sich um die Vorbeugung und/oder Behandlung von Verdauungsstörungen handelt.

14. Arzneimittel zur Anwendung in der Vorbeugung und/oder Behandlung von Lipasemangelerkrankungen gemäß einem der Ansprüche 12 oder 13,
**dadurch gekennzeichnet,**
**dass** es sich um die Vorbeugung und/oder Behandlung von exokriner Pankreasinsuffizienz bei Patienten mit Mukoviszidose oder um die Behandlung von exokriner Pankreasinsuffizienz von Patienten in der Pädiatrie handelt.

15. Verfahren zur Herstellung einer Schmelztablette gemäß einem der Ansprüche 1 bis 10, umfassend die Schritte:
- Bereitstellen einer wässrigen Lösung enthaltend die Burlulipase und Hilfsstoffe,
- Einfüllen der wässrigen Lösung in eine Form,
- Gefrieren der wässrigen Lösung in der Form,
- Gefriertrocknen der wässrigen Lösung in der Form.

## Claims

1. Orodispersible tablet, **characterized in that** it contains burlulipase.

2. Orodispersible tablet according to claim 1, **characterized in that** it contains a lyophilizate.

3. Orodispersible tablet according to one of claims 1 or 2, **characterized in that** it contains a lyophilizate of an aqueous solution of burlulipase.

4. Orodispersible tablet according to any one of claims 1 to 3, **characterized in that** it contains a lyophilizate of an aqueous solution of burlulipase which has been freeze-dried at a temperature of 0°C or less.

5. Orodispersible tablet according to one of claims 1 to 4, **characterized in that** it contains no effervescent composition and no disintegrants.

6. Orodispersible tablet according to any one of claims 1 to 5, **characterized in that** it contains at least one auxiliary substance which is selected from the group consisting of binders and structure-forming auxiliary substances.

7. Orodispersible tablet according to any one of claims 1 to 6, **characterized in that** it comprises burlulipase, binders, structure-forming auxiliary substances and optionally an acid or base to adjust the pH value and / or a surfactant to improve the disintegration or dissolution behavior.

8. Orodispersible tablet according to claim 7, **characterized in that** it contains burlulipase, fish gelatine, mannitol and sodium hydroxide.

9. Orodispersible tablet according to any one of claims 1 to 8, **characterized in that** it contains 0.1 to 20 mg burlulipase protein.

10. Orodispersible tablet according to any one of claims 1 to 8, **characterized in that** it contains 0.1 to 20 mg, preferably 0.5 to 10 mg and very particularly preferably 1 to 5 mg of burlulipase protein in a 200 µl blister, or **characterized in that** it contains between 5 and 25 mg of burlulipase protein in a 1200 µl blister.

11. Liquid pharmaceutical composition comprising burlulipase, **characterized in that** it is produced by introducing an orodispersible tablet according to any one of claims 1 to 10 into a liquid, **characterized in that** the orodispersible tablet comprises at least one binder.

12. Medicament for use in the prevention and / or treatment of lipase deficiency diseases comprising an orodispersible tablet according to one of claims 1 to 10 or a liquid pharmaceutical composition according to claim 11.

13. Medicament for use in the prevention and / or treatment of lipase deficiency diseases according to claim 12,
**characterized in that**
it refers to the prevention and / or treatment of digestive disorders.

14. Medicament for use in the prevention and / or treatment of lipase deficiency diseases according to one of claims 12 or 13,
**characterized in that**
it refers to the prevention and / or treatment of exocrine pancreatic insufficiency in patients with cystic fibrosis or to the treatment of exocrine pancreatic insufficiency in pediatric patients.

15. Method for producing an orodispersible tablet according to any one of claims 1 to 10, comprising the following steps:
- providing an aqueous solution containing the burlulipase and auxiliary substances,
- pouring the aqueous solution into a mold,
- freezing the aqueous solution in the mold,
- freeze-drying the aqueous solution in the mold.

## Revendications

1. Comprimé orodispersible, **caractérisé en ce qu'**il contient de la burlulipase.

2. Comprimé orodispersible selon la revendication 1, **caractérisé en ce qu'**il contient un lyophilisat.

3. Comprimé orodispersible selon l'une des revendications 1 ou 2, **caractérisé en ce qu'**il contient un lyophilisat d'une solution aqueuse de burlulipase.

4. Comprimé orodispersible selon l'une des revendications 1 à 3, **caractérisé en ce qu'**il contient un lyophilisat d'une solution aqueuse de burlulipase qui a été lyophilisée à une température inférieure ou égale à 0 °C.

5. Comprimé orodispersible selon l'une des revendications 1 à 4, **caractérisé en ce qu'**il ne comprend pas de préparation d'effervescence et pas d'agents désintégrants.

6. Comprimé orodispersible selon l'une des revendications 1 à 5, **caractérisé en ce qu'**il contient un moins un excipient qui est sélectionné dans le groupe constitué de liants et d'excipients de structuration.

7. Comprimé orodispersible selon l'une des revendications 1 à 6, **caractérisé en ce qu'**il comprend de la burlulipase, des liants, des excipients de structuration et, éventuellement, un acide ou une base destiné(e) à régler le pH et/ou un agent tensioactif destiné à améliorer la désintégration et/ou le processus de dissolution.

8. Comprimé orodispersible selon la revendication 7, **caractérisé en ce qu'**il contient de la burlulipase, de la gélatine de poisson, du mannitol et de l'hydroxyde de sodium.

9. Comprimé orodispersible selon l'une des revendications 1 à 8, **caractérisé en ce qu'**il contient 0,1 à 20 mg de burlulipase-protéine.

10. Comprimé orodispersible selon l'une des revendications 1 à 8, **caractérisé en ce qu'**il contient, dans un blister de 200 µL, 0,1 à 20 mg, de préférence 0,5 à 10 mg et, avec une préférence toute particulière, 1 à 5 mg de burlulipase-protéine, ou **caractérisé en ce qu'**il contient, dans un blister de 1200 µL, entre 5 et 25 mg de burlulipase-protéine.

11. Composition pharmaceutique liquide comprenant de la burlulipase, **caractérisée en ce qu'**elle est préparée en introduisant un comprimé orodispersible selon l'une des revendications 1 à 10 dans un liquide, **caractérisée en ce que** ledit comprimé orodispersible comprend au moins un liant.

12. Médicament destinée à être utilisé dans la prévention et/ou le traitement de maladies associées à un déficit en lipases, comprenant un comprimé orodispersible selon l'une des revendications 1 à 10 ou une composition pharmaceutique liquide selon la revendication 11.

13. Médicament destiné à être utilisé dans la prévention et/ou le traitement de maladies associées à un déficit en lipases selon la revendication 12,
**caractérisé en ce**
**qu'**il s'agit de prévenir et/ou traiter des troubles digestifs.

14. Médicament destiné à être utilisé dans la prévention et/ou le traitement de maladies associées à un déficit en lipases selon l'une des revendications 12 ou 13,
**caractérisé en ce qu'**il
s'agit de prévenir et/ou traiter une insuffisance pancréatique exocrine chez des patients atteints de mucoviscidose ou de traiter une insuffisance pancréatique exocrine de patients en pédiatrie.

15. Procédé de fabrication d'un comprimé orodispersible selon l'une des revendications 1 à 10, comprenant les étapes consistant à :
- préparer une solution aqueuse contenant ladite burlulipase et des excipients
- introduire la solution aqueuse dans un moule,
- congeler la solution aqueuse dans le moule,
- lyophiliser la solution aqueuse dans le moule.
